# EUROPÄISCHE PATENTSCHRIFT

(11) **EP 0 647 615 B1**
(45) Veröffentlichungstag und Bekanntmachung des Hinweises auf die Patenterteilung: **28.05.1997**
(21) Anmeldenummer: 94114856.1
(22) Anmeldetag: 21.09.1994
(51) Int. Cl.: C07C 227/08

(54) **Verfahren zur Herstellung von 5-Fluoranthranilsäurealkylestern und/oder 5-Fluoranthranilsäure**
Process for the preparation of 5-fluoroanthranilic acid alkyl esters and or 5-fluoroanthranilic acid
Procédé pour la préparation de l'acide 5-fluoroanthranilique et/ou ses esters alkyliques

(30) Priorität: 08.10.1993 DE 4334432
(43) Veröffentlichungstag der Anmeldung: 12.04.1995
(73) Patentinhaber: HOECHST AKTIENGESELLSCHAFT, 65929 Frankfurt am Main (DE)
(72) Erfinder: Rapp, Jochen, Dr., D-60322 Frankfurt am Main (DE); Planker, Siegfried, Dr., D-61462 Königstein (DE); Papenfuhs, Theodor, Dr., D-60433 Frankfurt am Main (DE); Bartels, Günter, Dr., D-30938 Burgwedel (DE)

(56) Entgegenhaltungen:
- DE-A- 2 345 788
- FR-A- 2 330 669
- JOURNAL OF THE AMERICAN CHEMICAL SOCIETY, Bd.66, Juli 1944, WASHINGTON, DC US Seiten 1165 - 1166 L.S. FOSDICK ET AL. 'The Synthesis of Some Alkyl and Dialkylaminoalkyl Esters of 2-Nitro-5-fluorobenzoic Acid and 2-Amino-fluorobenzoic Acid'
- TETRAHEDRON LETTERS, Bd.29, Nr.40, 1988, OXFORD GB Seiten 5105 - 5108 J.D. FARMER ER AL. 'SYNTHESIS AND DNA CROSSLINKING ABILITY OF A DIMERIC ANTHRAMYCIN ANALOG'

## Beschreibung

Die vorliegende Erfindung betrifft ein neues, vorteilhaftes Verfahren zur Herstellung von 5-Fluoranthranilsäurealkylestern und/oder 5-Fluoranthranilsäure.

Es ist bekannt, 5-Fluoranthranilsäure durch Nitrierung von 3-Fluorbenzoesäure und anschließende Reduktion der gebildeten 5-Fluor-2-nitrobenzoesäure herzustellen (Rec. Trav. Chim. Pays-Bas 1914, 33, 336, Tetrahedron Lett. 1988, 29(40), 5105-8; J. Biol. Chem. 1954, 207, 411-2).

Bei dieser Synthese bildet sich im Verlauf der Nitrierung stets auch die isomere 3-Fluor-2-nitrobenzoesäure, die sich jedoch von der 5-Fluor-2-nitrobenzoesäure nicht abtrennen läßt. Durch die nachfolgende Reduktion entsteht aus diesem Produktgemisch, das etwa 2 bis 3 % 3-Fluor-2-nitrobenzoesäure enthält, neben dem gewünschten Wertprodukt (5-Fluoranthranilsäure) im entsprechenden Umfange die 3-Fluoranthranilsäure, die sich allerdings nicht von der 5-Fluoranthranilsäure abtrennen läßt.

5-Fluoranthranilsäurealkylester lassen sich herstellen, indem man 5-Fluor-2-nitrobenzoesäure mittels Thionylchlorid in das entsprechende Säurechlorid überführt, dieses Säurechlorid anschließend mit einem Alkylalkohol zu einem 5-Fluor-2-nitrobenzoesäurealkylester umsetzt und diesen Alkylester mittels einer katalytischen Reduktion in den entsprechenden 5-Fluoranthranilsäurealkylester umwandelt (J. Am. Chem. Soc. 1944, 66, 1165-1166). Es ist bekannt, daß Nitrobenzoylchloride thermisch labil sind und zu Zersetzungsreaktionen neigen. Sie lassen sich daher nicht mittels Destillation aufarbeiten und im erforderlichen Umfange reinigen. Aus diesem Grund erweist sich eine technische Nutzung des Syntheseweges als recht problematisch und würde einen sehr hohen Sicherheitsaufwand erfordern.

DE-OS 23 45 778 betrifft ein Verfahren zur Herstellung eines Alkylhalogennitrobenzoats, insbesondere Methyl-5-chlor-2-nitrobenzoat (5-Chlor-2-nitrobenzoesäuremethylester), durch Nitrierung eines Alkylhalogenbenzoats in Gegenwart von Schwefelsäure und Salpetersäure. Hierbei verwendet man ein Gemisch aus Salpetersäure und Schwefelsäure und setzt diese Säuremischung der Reaktionszone, in der sich das umzusetzende Alkylhalogenbenzoat befindet, zu. Als Beispiel für einen Halogenbenzoesäurealkylester wird unter anderem auch Methyl-5-Fluor-2-nitrobenzoat (5-Fluor-2-nitrobenzoesäuremethylester) erwähnt.

Die Durchführung der Nitrierung wird allerdings nur durch ein einziges Beispiel, nämlich durch die Umsetzung von Methyl-5-chlor-2-nitrobenzoat (5-Chlor-2-nitrobenzoesäuremethylester) experimentell belegt. Man löst Methyl-3-chlorbenzoat in 1,2-Dichlorethan, kühlt die Lösung auf -10 °C ab und setzt eine Mischung aus Salpetersäure und Schwefelsäure langsam zu. Die Ausbeute an 5-Chlor-2-nitrobenzoesäuremethylester beträgt 85 Mol-%, die Reinheit lediglich 80 Gew.-%, was gegenüber dem in 94 Gew.-%iger Reinheit eingesetzten Methyl-3-chlorbenzoat eine deutliche Verschlechterung darstellt.

5-Fluoranthranilsäure, insbesondere isomerenreine 5-Fluoranthranilsäure, und 5-Fluoranthranilsäurealkylester stellen wertvolle Vorprodukte für die Herstellung von Pharmazeutika (Cz. P. 159 570), Herbiziden (US-PS 3 905 800, EP-PS 0 109 575, US-PS 4 388 472), Pflanzenwachstums-Regulatoren (Fr.-PS 2 541 288) und Fungiziden vom Chinazolintyp (US-PS 4 824 469) dar. Daher besteht ein erhebliches Interesse, diese Verbindungen technisch auf einfache Weise unter Berücksichtigung sicherheitstechnischer Belange zugänglich zu machen. Zudem soll das Verfahren sicherstellen, daß die gewünschten Produkte nicht nur in hoher Ausbeute, sondern auch in sehr hoher Reinheit anfallen.

Gelöst wird diese Aufgabe durch ein Verfahren zur Herstellung von 5-Fluoranthranilsäurealkylestern und/oder 5-Fluoranthranilsäure. Es ist dadurch gekennzeichnet, das man einen 3-Fluorbenzoesäurealkylester in Schwefelsäure löst und mit Nitriersäure bei -10 bis 30 °C umsetzt, anschließend Wasser zusetzt, das nitrierte Reaktionsprodukt abtrennt und in Gegenwart eines ein Metall der Platingruppe und Schwefel enthaltenden Katalysators mit Wasserstoff bei 50 bis 120 °C unter erhöhten Druck umsetzt und gegebenenfalls 5-Fluoranthranilsäurealkylester destillativ abtrennt und zu 5-Fluoranthranilsäure verseift.
Dem Verfahren liegt die nachfolgende Reaktionsgleichung zugrunde:

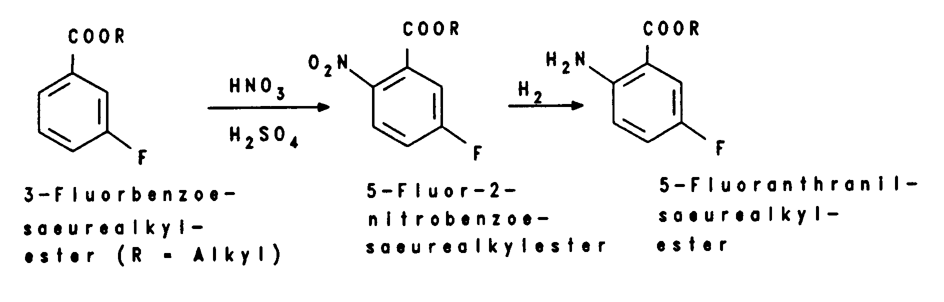

Als 3-Fluorbenzoesäuralkylester setzt man üblicherweise einen 3-Fluorbenzoesäure (C₁-C₆)alkylester ein.
Geeignete 3-Fluorbenzoesäurealkylester sind 3-Fluorbenzoesäuremethylester, 3-Fluorbenzoesäureethylester, 3-Fluorbenzoesäure-n-propylester, 3-Fluorbenzoesäure-i-propylester, 3-Fluorbenzoesäure-n-butylester und/oder 3-Fluorbenzoesäure-i-butylester.
Gut geeignet als Ausgangsstoff sind 3-Fluorbenzoesäuremethylester und/oder 3-Fluorbenzoesäuerethylester.
In einer Reihe von Fällen kann es vorteilhaft sein, als 3-Fluorbenzoesäurealkylester den 3-Fluorbenzoesäuremethylester zu verwenden.
Man löst den 3-Fluorbenzoesäurealkylester in Schwefelsäure, wobei darauf geachtet werden sollte, daß die Temperaturen hierbei nicht zu hoch gewählt werden. Im allgemeinen löst man den 3-Fluorbenzoesäurealkylester bei 0 bis 20 °C in Schwefelsäure auf. Es ist allerdings möglich das Auflösen bei höheren Temperaturen, beispielsweise bis 40 °C, vorzunehmen.
Die für das Auflösen verwendete Schwefelsäure soll möglichst keinen Wasseranteil aufweisen, sondern in konzentrierter Form Anwendung finden.
Üblicherweise löst man den 3-Fluorbenzoesäurealkylester in 98 bis 100 Gew-%iger H₂SO₄ auf.
Bei Durchführung des erfindungsgemäßen Verfahrens ist darauf zu achten, daß man die Schwefelsäure in ausreichender Menge verwendet. Im allgemeinen genügt, es den 3-Fluorbenzoesäurealkylester in 2 bis 10, insbesondere 3 bis 6 Gew.-Teilen H₂SO₄, bezogen auf 3-Fluorbenzoesäurealkylester, zu lösen.
Der in Schwefelsäure gelöste 3-Fluorbenzoesäurealkylester wird anschließend mit Nitriersäure behandelt. Unter Nitriersäure wird ein Gemisch aus HNO₃ und H₂SO₄ verstanden, wobei sowohl die Salpetersäure als auch die Schwefelsäure in hochkonzentrierter Form, d.h. mit einem Wasseranteil von 0 bis 3 Gew.-% Wasser, verwendet werden.
Üblicherweise setzt man als Nitriersäure ein Gemisch aus 95 bis 100 Gew.-%iger HNO₃ und 98 bis 100 Gew.-%iger H₂SO₄ ein. Im allgemeinen verwendet man als Nitriersäure ein aus 1 Gew.-Teil HNO₃ und 2 bis 6 Gew.-Teilen H₂SO₄ bestehendes Gemisch.

Die Nitrierung des 3-Fluorbenzoesäurealkylesters läßt sich sowohl mit einem Unterschuß als auch mit einem Überschuß an Nitriersäure durchführen.
Üblicherweise setzt man je Mol 3-Fluorbenzoesäurealkylester 0,8 bis 1,2 Äquivalente Nitriersäure (bezogen auf enthaltene Salpetersäure) ein. In einer Reihe von Fällen kann es günstig sein, je Mol 3-Fluorbenzoesäurealkylester 0,95 bis 1,05 Äquivalente Nitriersäure (bezogen auf enthaltene Salpetersäure) einzusetzen. Besonders einfach gestaltet sich die Umsetzung, indem man die Nitriersäure in äquivalenter Menge, bezogen auf 3-Fluorbenzoesäurealkylester, zusetzt.

Bei der Umsetzung mit Nitriersäure sollten allzu hohe Temperaturen vermieden werden. Im allgemeinen genügt es, die Nitrierung bei -10 bis 30 °C durchzuführen. Für eine Reihe von Fällen erweist sich ein Temperaturbereich von 0 bis 30, insbesondere 0 bis 20 °C als ausreichend.

Nach Beendigung der Nitrierung wird das anfallende Reaktionsgemisch mit Wasser versetzt. Während des Verdünnens mit Wasser sollte die Temperatur nicht zu hoch ansteigen. Es empfiehlt sich, gegebenenfalls das Reaktionsgemisch und/oder das zugesetzte Wasser zu kühlen. Im allgemeinen sollte die Wasserzugabe so erfolgen, daß ein Temperaturbereich von etwa 40 bis etwa 60 °C eingehalten wird.

Zweckmäßigerweise verdünnt man das nach der Nitrierung anfallende Reaktionsgemisch mit Wasser soweit, bis daß der 5-Fluor-2-nitrobenzoesäurealkylester, der in untergeordneten Mengen noch 3-Fluor-2-nitrobenzoesäurealkylester enthält, sich abscheidet. Besonders einfach gestaltet sich das Verfahren, wenn man hierbei eine Temperatur wählt, bei der sich der Nitroester in flüssiger Form abtrennen läßt. Man trennt die organische, das Wertprodukt enthaltende Phase von der wäßrigen, Schwefelsäure enthaltenden Phase. Die wäßrige Phase läßt sich mittels technisch üblicher Verfahren zu wiedereinsetzbarer 100 %iger Schwefelsäure regenerieren. Die organische Phase wird, gegebenenfalls nach Waschen mit 40 bis 60 °C warmen Wasser in die nachfolgende Reduktion eingesetzt.
Es ist als überraschend anzusehen, daß die Nitrierung bereits ein Wertprodukt in hoher Reinheit liefert. Man findet neben dem gewünschten 5-Fluor-2-nitrobenzoesäurealkylester nicht mehr als etwa 2 Gew.-% oder weniger an unerwünschtem 3-Fluor-2-nitrobenzoesäurealkylester. Dies dürfte hinsichtlich der in der DE-OS 23 45 788 experimentell belegten Nitrierung von 3-Chlorbenzoesäuremethylester, die zu einer deutlichen Herabsetzung der Reinheit des Wertproduktes führt, als unerwartetes Resultat anzusehen sein.
In diesem Zusammenhang ist jedoch darauf hinzuweisen, daß ein Anteil von etwa 2 Gew.-% an 3-Fluor-2-nitrobenzoesäurealkylester eine für die weitere Verwendung von 5-Fluor-2-benzoesäurealkylester nicht tolerierbare Größenordnung darstellt.

Ein wesentlicher Vorteil des erfindungsgemäßen Verfahrens besteht darin, daß es sich, ohne Abstriche hinsichtlich der Reinheit von 5-Fluoranthranilsäureester und/oder 5-Fluoranthranilsäure in Kauf nehmen zu müssen, flexibel anwenden läßt. Es eröffnet nämlich die Möglichkeit, je nach Wunsch und Bedarf entweder das nach der Nitrierung anfallende Reaktionsgemisch ohne eine zusätzliche Reinigung in die Reduktion einzusetzen oder aber auf vergleichsweise einfache Art und Weise den unerwünschten 3-Fluor-2-nitrobenzoesäurealkylester nahezu vollständig abzutrennen und den derart erhaltenen isomerenfreien 5-Fluor-2-nitrobenzoesäurealkylester der Reduktion zuzuführen. Darüberhinaus besteht die Möglichkeit, den isomerenfreien 5-Fluor-2-nitrobenzoesäurealkylester mittels Hydrolyse in eine isomerenfreie 5-Fluor-2-nitrobenzoesäure zu überführen und diese anschließend zu reduzieren. Nach Reduktion des isomerenfreien Nitrierproduktes erhält man nach Wahl einen isomerenfreien 5-Fluoranthranilsäurealkylester oder eine isomerenfreie 5-Fluoranthranilsäure. Falls gewünscht, lassen sich auch Mischungen dieser beiden Stoffe herstellen.

Die Reinigung des 3-Fluor-2-nitrobenzoesäurealkylester als Verunreinigung enthaltenden Reaktionsgemisches gelingt mit vergleichbar niedrigen Aufwand durch Schmelzkristallisation. Bei der Schmelzkristallisation wird das in flüssiger Form vorliegende Nitrierprodukt durch Abkühlen vollständig zur Kristallisation gebracht und anschließend durch sehr langsames Aufheizen 0,5 bis 2 °C pro Stunde soweit erwärmt, bis sich flüssige Anteile bilden, die aus dem Feststoff (Rest-Regulus) mittels technisch geeigneter Vorrichtungen (Abtropf-Apparat, Kristallisierkolonnen) abgetrennt werden können. Die flüssigen Anteile enthalten vorzugsweise den 3-Fluor-2-nitrobenzoesäurealkylester, während das Wertprodukt (5-Fluor-2-nitrobenzoesäurealkylester) im Rest-Regulus verbleibt.
Die Reinigungsoperation wird durch analytische Überwachung der abgeschmolzenen Fraktionen überwacht. Sobald die abgeschmolzene Fraktion isomerenreinen 5-Fluor-2-nitrobenzoesäurealkylester ausweist, ist die Reinigung abgeschlossen und der Rest-Regulus wird durch forciertes Aufschmelzen verflüssigt und der Weiterverarbeitung zugeführt. Die zuvor gewonnen Fraktionen ("Tropföle") können ganz oder teilweise einer nachfolgenden Schmelzkristallisation zugeführt werden.
Die Hydrolyse der auf diese Weise isomerenrein erhaltenen 5-Fluor-2-nitrobenzoesäurealkylester kann sowohl im alkalischen als auch im sauren Medium erfolgen. Im allgemeinen ist einer sauren Hydrolyse, beispielsweise unter Verwendung einer wäßrigen Mineralsäure, den Vorzug zu geben.

Zur Weiterverarbeitung setzt man das nitrierte Reaktionsprodukt, wie eingangs bereits erwähnt, in Gegenwart eines ein Metall der Platingruppe und Schwefel enthaltenden Katalysators mit Wasserstoff um. Üblicherweise setzt man 0,01 bis 0,1 Gew.-% des Metalles der Platingruppe, bezogen auf nitriertes Reaktionsprodukt, ein.
Geeignet als Katalysator sind solche, die Pd oder Pt und Schwefel enthalten. Die Reduktion gestaltet sich unter Verwendung von Trägerkatalysatoren, insbesondere Pd oder Pt enthaltenden Trägerkatalysatoren, besonders einfach.
Gut geeignet als Katalysator sind solche, die Pd oder Pt auf Aktivkohle als Träger und untergeordnete Mengen einer Schwefelverbindung enthalten. Die Schwefelverbindung kann man dem Katalysator separat zusetzen, sie kann jedoch auch im Katalysator vorhanden sein und beispielsweise in Form eines sulfitierten oder sulfidierten Katalysators, gegebenenfalls in Anwesenheit einer weiteren Schwefelverbindung, Anwendung finden.
Der Katalysator enthält untergeordnete Mengen der Schwefelverbindung.
Üblicherweise setzt man Schwefelverbindung und Platinmetall im Verhältnis (0,05 bis 40) zu 1, insbesondere (0,2 bis 10) zu 1, ein. Geeignete Schwefelverbindungen sind Thioharnstoff, Dimethylsulfoxid, Thiophen und/oder ein Alkalisulfit.
Für die Umsetzung mit Wasserstoff empfiehlt es sich, das nitrierte Reaktionsprodukt in Gegenwart eines inerten Lösungsmittels zur Reaktion zu bringen. Als geeignetes inertes Lösungsmittel kann man H₂O, einen Alkohol, insbesondere einen aliphatischen Alkohol mit 1 bis 5 Kohlenstoffatomen, Toluol, Xylol, Gemische isomerer Xylole, Chlorbenzol und/oder Dichlorbenzol verwenden.

Beabsichtigt man, eine isomerenreine 5-Fluor-2-nitrobenzoesäure mit Wasserstoff umzusetzen, so ist es günstig, eine wäßrige Lösung eines Salzes dieser isomerenreinen 5-Fluor-2-nitrobenzoesäure als nitriertes Reaktionsprodukt zu gebrauchen.
Man führt die Umsetzung mit Wasserstoff unter Druck durch, wobei ein Druck von 0,5 bis 10, insbesondere 1,0 bis 3,0 MPa ausreichend ist. Wie eingangs bereits erwähnt, führt man die Reduktion mit Wasserstoff bei 50 bis 120 °C durch. Für eine Vielzahl von Fällen hat es sich als ausreichend erwiesen, diese Umsetzung bei 60 bis 100 °C durchzuführen. Nach Beendigung der Reduktion wird der Katalysator abfiltriert und das im Lösungsmittel gelöste Produkt von der gebildeten Wasserphase getrennt. Die organische Phase wird durch Destillation vom Lösungsmittel befreit, wobei der 5-Fluoranthranil-säurealkylester als Sumpfprodukt anfällt und, falls erforderlich, destillativ gereinigt wird.
Falls ein isomerenhaltiger Nitroester in die Reduktion eingesetzt wird, kann der 3-Fluoranthranilsäurealkylester als Nebenprodukt enthaltende 5-Fluoranthranilsäurealkylester durch fraktionierte Destillation gereinigt werden, wobei 3-Fluoranthranilsäurealkylester als Vorlauf abgetrennt und der 5-Fluoranthranilsäurealkylester anschließend als Sumpfprodukt oder nach weiterer Destillation als isomerenreines Produkt gewonnen wird. Falls gewünscht, kann man den isomerenreinen 5-Fluoranthranilsäurealkylester durch Hydrolyse in die 5-Fluoranthranilsäure überführen. Es ist daher nicht unbedingt erforderlich, bereits das rohe nitrierte Reaktionsprodukt einer Reinigung zu unterwerfen, um zu einem isomerenreinen 5-Fluoranthranilsäurealkylester und/oder isomerenreiner 5-Fluoranthranilsäure zu gelangen. Der entsprechende Reinigungsschritt kann auch zu einem späteren Zeitpunkt, nämlich nach Beendigung der Reduktion durchgeführt werden. Dadurch erweist sich das erfindungsgemäße Verfahren in besonderer vorteilhafter Weise als variabel und läßt sich den jeweiligen Wünschen entsprechend angepaßt ausführen. Es liefert nämlich nicht nur zum einen isomerenreinen 5-Fluoranthranilsäurealkylester und zum anderen isomerenreine 5-Fluoranthranilsäure, sondern auch, je nach Bedarf, isomerenreinen 5-Fluor-2-nitrobenzoesäurealkylester und isomerenreine 5-Fluor-2-nitrobenzoesäure und Mischungen dieser Verbindungen.

Die nachfolgenden Beispiele belegen die Erfindung, ohne sie zu beschränken.

### Experimenteller Teil

### Beispiel 1

In 2025 Teilen 100 %iger Schwefelsäure werden unter Rühren und leichtem Kühlen bei maximal 35 °C 504 Teile 3-Fluorbenzoesäureethylester (Reingehalt 99,7 Gew.-%) gelöst. Dann tropft man bei 10 - 20 °C in 90 Minuten eine Mischung aus 210 Teilen Salpetersäure 98 %ig und 420 Teilen Schwefelsäure 100 %ig zu und rührt anschließend eine Stunde ohne Kühlung nach.
Nun läßt man die Nitriermischung so zu vorgelegten 995 Teilen Eiswasser zulaufen, daß 50 °C nicht überschritten werden, trennt den sich flüssig abscheidenden Fluor-nitrobenzoesäureethylester ab und wäscht ihn in 3 Portionen mit 200 Teilen 50 °C warmem Wasser neutral.
Zur Reduktion kann das Produkt nach Wassergehaltsbestimmung feucht eingesetzt werden (vgl. Beispiel 5), zur Isomerenabtrennung durch Schmelzkristallisation (vgl. Beispiel 3) wird es bei 100 °C im Vakuum getrocknet. Man erhält 580 Teile Fluor-nitrobenzoesäureethtlester, bestehend aus 98,2 % 5-Fluor-2-nitrobenzoesäureethylester und 1,8 % 3-Fluor-2-nitrobenzoesäureethylester, mit einem Erstarrungspunkt Ep. 44,6 °C und einem Reingehalt (GC) von > 99 %, bezogen auf Isomerengemisch. Dies entspricht einer Ausbeute von 90 % d. Th., bezogen auf eingesetzten 3-Fluorbenzoesäureethylester.

### Beispiel 2

Man löst 72,4 Teile 3-Fluorbenzoesäuremethylester bei maximal 30 °C unter Rühren in 300 Teilen 100 %iger Schwefelsäure, kühlt auf 0-10 °C ab und tropft dann im Verlaufe von 2 Stunden eine Mischung aus 32,7 Teilen Salpetersäure 98 %ig und 78,3 Teilen Schwefelsäure 100 %ig unter ständiger Kühlung bei 0 - 10 °C zu, rührt 3 Stunden ohne Kühlung nach, wobei die Temperatur der Nitriermischung bis auf 20° C ansteigt. Man tropft nun, wiederum unter ständiger Kühlung, 175 Teile Wasser zu. Dabei scheidet sich der Fluor-nitrobenzoesäuremethylester als Öl ab, das abgetrennt, mit 350 Teilen Wasser portionsweise neutralgewaschen und im Vakuum bei 100 °C getrocknet wird.
Man erhält 92 Teile Fluor-nitrobenzoesäure-methylester, bestehend aus 98,1 % 5-Fluor-2-nitro-benzoesäuremethylester und 1,9 % 3-Fluor-2-nitrobenzoesäuremethylester, vom Ep. 34,0 °C und Reingehalt (GC) von > 99 %, bezogen auf Isomerengemisch. Dies entspricht einer Ausbeute von 98,4 % d. Th., bezogen auf eingesetzten 3-Fluorbenzoesäuremethylester.

### Beispiel 3

1295,8 Teile gemäß Beispiel 1 hergestellter trockener Fluor-nitro-benzoesäureethylester (Isomerengemisch aus 98,2 % = 1272,5 Teilen 5-Fluor-2-nitrobenzoesäureethylester und 1,8 % = 23,3 Teilen 3-Fluor-2-nitrobenzoesäureethylester) vom Ep. 44,6° C werden schmelzflüssig in einen Tropfapparat (beheiztes, senkrecht stehendes Glasrohr mit verschließbarem Bodenauslauf, Durchmesser 5 cm, Höhe 60 cm) eingefüllt und über Nacht durch Abkühlen auf 20 °C vollständig zur Kristallisation gebracht.
Am nächsten Morgen heizt man den Tropfapparat kontinuierlich mit einer Heizrate von 1 °C/Stunde bei geöffnetem Bodenauslauf auf und untersucht die abtropfenden Fraktionen gaschromatographisch auf die aktuelle Isomeren-Zusammensetzung. (Fraktionenbilanz in nachfolgender Tabelle).
Bei 34 °C beginnt geschmolzenes, an 3-Fluor-2-nitrobenzoesäureethylester angereichertes Produkt abzutropfen. Wenn eine Temperatur von 44,5 °C erreicht ist, sind ca. 25 % des Einsatzproduktes abgetropft, die 98 % des unerwünschten Isomeren enthalten. Der im Tropfapparat verbleibende Rest-Regulus ist isomerenreiner 5-Fluor-2-nitrobenzoesäure-ethylester vom Reingehalt 99,95 % (GC), Ep.: 45,6° C.

### Fraktionenbilanz

| Fraktion | Temp. (°C) | Menge (g) | 3-Fluor-2-nitrobenzoesäureethylester | | | % v. Einsatz (aufsummiert) |
|---|---|---|---|---|---|---|
| | | | Gehalt (%) | Menge (g) | Σ Menge (g) | |
| 1 | 34-36 | 18,3 | 31,1 | 5,7 | 5,7 | 1,4 |
| 2 | 36-38 | 17,3 | 18,5 | 3,2 | 8,9 | 2,7 |
| 3 | 38-40 | 36,1 | 14,4 | 5,2 | 14,1 | 5,5 |
| 4 | 40-42 | 8,9 | 9,0 | 0,8 | 14,9 | 6,2 |
| 5 | 42-43 | 14,1 | 6,4 | 0,9 | 15,8 | 7,3 |
| 6 | 43-43,5 | 42,4 | 4,5 | 1,9 | 17,7 | 10,6 |
| 7 | 43,5 | 27,6 | 4,0 | 1,1 | 18,8 | 12,7 |
| 8 | 43,5 | 42,5 | 4,0 | 1,7 | 20,5 | 16,0 |
| 9 | 43,5-44 | 68,4 | 2,3 | 1,6 | 22,1 | 21,3 |
| 10 | 44-44,5 | 43,9 | 1,8 | 0,8 | 22,9 | 24,7 |

Die Ausbeute an isomerenreinem 5-Fluor-2-nitrobenzoesäure-ethylester beträgt somit 75,3 %, bezogen auf eingesetztes Isomerengemisch.
Durch Rückführung der Fraktionen 4 - 10 in die nächste Schmelzkristallisation läßt sich die Ausbeute an Reinester auf 89 - 91 % steigern.

### Beispiel 4

213 Teile isomerenreiner 5-Fluor-2-nitrobenzoesäureethylester, hergestellt durch Schmelzkristallisation gemäß Beispiel 3, wird nach Zusatz von 912 Teilen 20 %iger Salzsäure und 600 Teilen Eisessig 20 Stunden auf 100 °C erhitzt, wobei im Zuge der Verseifung 545 Teile wäßriges Ethanol abdestillieren.
Anschließend kühlt man auf Raumtemperatur und isoliert die dabei ausfallende 5-Fluor-2-nitrobenzoesäure auf einer Nutsche. Der Filterkuchen wird mit 300-400 Teilen Wasser neutralgewaschen. Das Mutterfiltrat wird auf ca. 150 Teile eingeengt. Beim Abkühlen fällt weitere 5-Fluor-2-nitrobenzoesäure aus, die nach Abfiltrieren und Waschen mit der Erstfällung vereinigt wird.
Man erhält 230 Teile Feuchtprodukt (Wassergehalt 21,3 %), das als solches zur Reduktion eingesetzt werden kann (vgl. Beispiel 7).
Durch Trocknen bei 100 °C im Vakuum erhält man 181 Teile isomerenreine 5-Fluor-2-nitrobenzoesäure vom Schmelzpunkt 134 - 135 °C, was einer Ausbeute von 97,8 % d. Th. entspricht.

### Beispiel 5

Im Hydrierautoklav werden 344 Teile Xylol, 1 Teil Triethylamin, 4,5 Teile Katalysator (5 % Pt auf Kohle, wasserfeucht, Wassergehalt 50 %) und 0,05 Teile Natriumsulfit vorgelegt. Nach je dreimaligem Spülen des Gasraumes mit Stickstoff und Wasserstoff wird unter Rühren auf 80 - 85 °C aufgeheizt und bei einem Wasserstoffdruck von 1,5 - 2,0 MPa innerhalb 90 Minuten eine Lösung aus 400 Teilen 5-Fluor-nitrobenzoesäure-ethylester (Isomerengemisch, hergestellt gemäß Beispiel 1, bestehend aus 98,2 % = 392,8 Teilen 5-Fluor-2-nitro-benzoesäureethylester und 1,8 % = 7,2 Teilen 3-Fluor-2-nitrobenzoesäureethylester) in 516 Teilen Xylol, der noch 1 Teil Triethylamin zugesetzt wurde, zugepumpt.
Wenn kein Druckabfall mehr erfolgt, rührt man noch 20 Minuten bei 85 - 90 °C nach, entspannt und filtriert den Katalysator bei 30 - 60 °C über ein Druckfilter ab.
Aus dem Filtrat wird die wäßrige Phase abgetrennt, das Xylol abdestilliert und der verbleibende rohe Fluoranthranilsäureethylester im Vakuum von 1 Torr fraktioniert. Nach einem 5 %igen Vorlauf (bezogen auf eingesetzes Rohprodukt), der den gesamten isomeren 3-Fluoranthranilsäureethylester enthält (Kp₁: 85 - 95 °C), destilliert reiner
5-Fluoranthranilsäureethylester über (Kp₁: 102 °C). Man erhält ihn in einer Ausbeute von 93,5 % d. Th., bezogen auf eingesetztes Isomerengemisch. Der Reingehalt (GC) liegt bei 99,9 %.

Ersetzt man die 0,05 Teile Natriumsulfit durch 0,01 Teile Thioharnstoff und das Triethylamin durch aliquote Mengen Morpholin, so erhält man bei gleicher Arbeitsweise ein identisches Ergebnis.

### Beispiel 6

Entsprechend Beispiel 5 werden 316 Teile Methanol, 1,5 Teile Triethylamin und 5 Teile Katalysator (5 % Pt auf Kohle, wasserfeucht, Wassergehalt 50 %) und 0,05 Teile Dimethylsulfoxid vorgelegt und bei einem Wasserstoffdruck von 1,0 bis 1,5 MPa bei 85 - 90 °C eine Lösung aus 348 Teilen 5-Fluor-nitrobenzoesäuremethylester (Isomerengemisch, hergestellt gemäß Beispiel 2, bestehend aus 98,1 % = 341,4 Teilen 5-Fluor-2-nitrobenzoesäuremethylester und 1,9 % = 6,6 Teilen 3-Fluor-2-nitrobenzoesäuremethylester) in 395 Teilen Methanol, der noch 0,5 Teile Triethylamin zugesetzt wurden, innerhalb 60 Minuten zugepumpt. Nach Reaktionsende wird der Katalysator durch Filtration abgetrennt und das Filtrat fraktioniert destilliert. Nach einem Vorlauf (Methanol/Wasser) wird bei 90 - 103 °C/1 Torr ein Zwischenlauf geschnitten, der den gesamten 3-Fuoranthranilsäuremethylester enthält (17,7 Teile = 6 %) und anschließend isomerenreiner 5-Fluoranthranilsäuremethylester (Kp₁: 104 °C) überdestilliert. Man erhält 272 Teile vom Reingehalt 99,9 %, was einer Ausbeute von 92 % d. Th., bezogen auf eingesetztes Isomerengemisch, entspricht.

### Beispiel 7

Entsprechend Beispiel 5 werden 400 Teile isomerenreiner 5-Fluor-2-nitrobenzoesäureethylester, hergestellt durch Schmelzkristallisation gemäß Beispiel 3, katalytisch reduziert.
Nach Abtrennen des Katalysators und der Wasserphase und Trocknen der organischen Phase im Vakuum von 100 Torr bei 100 °C erhält man den isomerenreinen 5-Fluoranthranilsäure-ethylester mit einem Reingehalt (GC) von 99,7 % in einer Ausbeute von 98,9 %.

Ersetzt man den isomerenreinen 5-Fluor-2-nitrobenzoesäureethylester durch aliquote Teile eines entsprechend hergestellten isomerenreinen 5-Fluor-2-nitrobenzoesäure-n-propylesters und arbeitet in der gleichen Weise, so erhält man den isomerenreinen 5-Fluoranthranilsäure-n-propylester in vergleichbarer Ausbeute und Qualität.

### Beispiel 8

Im Hydrierautoklav werden 450 Teile Wasser und 2,5 Teile Katalysator (5 % Pt auf Kohle, sulfitiert, wasserfeucht, Wassergehalt 50 %) vorgelegt. Nach je dreimaligem Spülen des Gasraumes mit Stickstoff und Wasserstoff wird unter Rühren auf 90 °C aufgeheizt und bei einem Wasserstoffdruck von 1,0 - 2,0 MPa innerhalb 60 Minuten eine Lösung von 231 Teilen isomerenreiner 5-Fluor-2-nitro-benzoesäure (hergestellt durch saure Verseifung von schmelzkristallisiertem 5-Fluor-2-nitrobenzoesäureethylester gemäß Beispielen 3 + 4) in 400 Teilen 12,5 %iger Natronlauge zugepumpt.
Wenn kein Druckabfall mehr erfolgt, rührt man noch 20 Minuten bei 90 °C nach, entspannt, filtriert vom Katalysator bei 40 - 60° C und säuert das wäßrige Filtrat mit Salzsäure an. Dabei fällt die isomerenreine 5-Fluoranthranilsäure aus. Sie wird abfiltriert, mit Wasser neutral gewaschen und im Vakuum bei 100 °C getrocknet.
Man erhält 179 Teile isomerenreine 5-Fluoranthranilsäure vom Schmelzpunkt 182-182,5 °C und einem Reingehalt von 99,7 % (HPLC), was einer Ausbeute von 92,4 % d. Th., bezogen auf eingesetzte isomerenreine 5-Fluor-2-nitrobenzoesäure, entspricht.

### Beispiel 9

183 Teile isomerenreiner 5-Fluoranthranilsäureethylester, hergestellt gemäß Beispiel 5, und 912 Teile 20 %ige Salzsäure werden 10 Stunden unter Rückfluß erhitzt. Dann wird das gebildete Ethanol und die Hauptmenge der wäßrigen Salzsäure abdestilliert. Der verbleibende Sumpf wird mit Natronlauge auf pH 4,0 eingestellt. Die dabei auskristallisierende isomerenreine 5-Fluoranthranilsäure wird abfiltriert, mit Wasser neutral gewaschen und im Vakuum bei 100 °C getrocknet.
Man erhält 149 Teile isomerenreine 5-Fluoranthranilsäure vom Schmelzpunkt 182-182,5 °C und einem Reingehalt von 99,8 % (HPLC), was einer Ausbeute von 96,1 % d. Th., bezogen auf eingesetzten isomerenreinen 5-Fluoranthranilsäureethylester, entspricht.

### Vergleichsbeispiel 1

### (Nitrierung von 3-Fluorbenzoesäuremethylester gemäß DOS 23 45 788, Beispiel 1)

Zu einer vorgelegten Lösung von 172,4 Teilen 3-Fluorbenzoesäuremethylester (Reingehalt 99,8 %) in 50 Teilen Methylenchlorid tropft man eine Mischung aus 33,8 Teilen 95 %iger Salpetersäure und 81,6 Teilen 98,6 %iger Schwefelsäure in 3 Stunden bei -10 °C zu. Anschließend wird 2 Stunden bei -10 °C und 3 Stunden bei 0-30 °C nachgerührt.
Der Fluor-nitrobenzoesäuremethylester wird dann mit 1,2-Dichlorethan aus der Reaktionsmischung extrahiert und durch Verdampfen des Lösungsmittels isoliert.

Man erhält 79,8 Teile Produkt mit folgender Zusammensetzung:
13,3 % = 10,6 Teile 3-Fluorbenzoesäuremethylester
84,4 % = 67,4 Teile 5-Fluor-2-nitrobenzoesäuremethylester
2,3 % = 1,8 Teile 3-Fluor-2-nitrobenzoesäuremethylester
d. h., der gewünschte 5-Fluor-2-nitrobenzoesäuremethylester wird in 85,3 %iger Ausbeute gebildet. Sein Reingehalt beträgt lediglich 84,4 %, das Isomerenverhältnis liegt mit 2,7 % 3-Fluor-2-nitrobenzoesäuremethylester zu 97,3 % 5-Fluor-2-nitrobenzoesäuremethylester schlechter, als beim erfindungsgemäßen Verfahren (1,8 - 1,9 % 3-Fluor-2-nitrobenzoesäuremethylester zu 98,1 - 98,2 % 5-Fluor-2-nitrobenzoesäuremethylester).

### Vergleichsbeispiel 2

### Katalytische Reduktion von 5-Fluor-nitrobenzoesäureethylester (Isomerengemisch, hergestellt nach Beispiel 1) in Abwesenheit von Moderatoren (Schwefelverbindungen)

Arbeitet man wie im Beispiel 5 angegeben, verzichtet aber auf den Zusatz der 0,05 Teile Natriumsulfit oder 0,01 Teile Thioharnstoff, so erhält man nach katalytischer Reduktion und destillativer Aufarbeitung einen 5-Fluoranthranilsäureethylester in vergleichbarer Ausbeute, der aber wegen eines Gehaltes von 0,3 - 0,4 % Anthranilsäureethylester (entstanden durch hydrogenolytische Fluorabspaltung) nicht für Folgereaktionen einsetzbar ist. Eine destillative Abtrennung dieser Verunreinigung ist selbst bei hohem Fraktionieraufwand nicht möglich, da sie den gleichen Siedepunkt wie der 5-Fluor-anthranilsäuremethylester hat.

## Patentansprüche

1. Verfahren zur Herstellung von 5-Fluoranthranilsäurealkylestern und/oder 5-Fluoranthranilsäure, dadurch gekennzeichnet, daß man einen 3-Fluorbenzoesäurealkylester in Schwefelsäure löst und mit Nitriersäure bei -10 bis 30 °C umzetzt, anschließend Wasser zusetzt, das nitrierte Reaktionsprodukt abtrennt und in Gegenwart eines ein Metall der Platingruppe und Schwefel enthaltenden Katalysators mit Wasserstoff bei 50 bis 120 °C unter erhöhten Druck umsetzt und gegebenenfalls 5-Fluoranthranilsäurealkylester destillativ abtrennt und zu 5-Fluoranthranilsäure verseift.

2. Verfahren nach Anspruch 1, dadurch gekennzeichnet, daß man als 3-Fluorbenzoesäurealkylester einen 3-Fluorbenzoesäure (C₁-C₆)alkylester einsetzt.

3. Verfahren nach Anspruch 1 oder 2, dadurch gekennzeichnet, daß man 3-Fluorbenzoesäuremethylester, 3-Fluorbenzoesäureethylester, 3-Fluorbenzoesäure-n-propylester, 3-Fluorbenzoesäure-i-propylester, 3-Fluorbenzoesäure-n-butylester und/oder 3-Fluorbenzoesäure-i-butylester einsetzt.

4. Verfahren nach einem oder mehreren der Ansprüche 1 bis 3, dadurch gekennzeichnet, daß man 3-Fluorbenzoesäuremethylester und/oder 3-Fluorbenzoesäureethylester einsetzt.

5. Verfahren nach einem oder mehreren der Ansprüche 1 bis 4, dadurch gekennzeichnet, daß man 3-Fluorbenzoesäuremethylester einsetzt.

6. Verfahren nach einem oder mehreren der Ansprüche 1 bis 5, dadurch gekennzeichnet, daß man den 3-Fluorbenzoesäurealkylester bei 0 bis 20 °C in H₂SO₄ löst.

7. Verfahren nach einem oder mehreren der Ansprüche 1 bis 6, dadurch gekennzeichnet, daß man den 3-Fluorbenzoesäurealkylester in 98 bis 100 Gew.- %iger H₂SO₄ löst.

8. Verfahren nach einem oder mehreren der Ansprüche 1 bis 7, dadurch gekennzeichnet, daß man den 3-Fluorbenzoesäurealkylester in konzentrierter H₂SO₄ löst.

9. Verfahren nach einem oder mehreren der Ansprüche 1 bis 8, dadurch gekennzeichnet, daß man den 3-Fluorbenzoesäurealkylester in 2 bis 10 Gew.-Teilen H₂SO₄, bezogen auf 3-Flourbenzoesäurealkylester, löst.

10. Verfahren nach einem oder mehreren der Ansprüche 1 bis 9, dadurch gekennzeichnet, daß man als Nitriersäure ein Gemisch aus 95 bis 100 Gew.-%iger HNO₃ und 98 bis 100 Gew.- %iger H₂SO₄ einsetzt.

11. Verfahren nach einem oder mehreren der Ansprüche 1 bis 10, dadurch gekennzeichnet, daß man als Nitriersäure ein aus 1 Gew.-Teil HNO₃ und 2 bis 6 Gew.-Teilen konzentrierter H₂SO₄ bestehendes Gemisch einsetzt.

12. Verfahren nach einem oder mehreren der Ansprüche 1 bis 11, dadurch gekennzeichnet, daß man je Mol 3-Fluorbenzoesäurealkylester 0,8 bis 1,2 Äquivalente Nitriersäure (bezogen auf enthaltene Salpetersäure) einsetzt.

13. Verfahren nach einem oder mehreren der Ansprüche 1 bis 12, dadurch gekennzeichnet, daß man je Mol 3-Fluorbenzoesäurealkylester 0,95 bis 1,05 Äquivalente Nitriersäure (bezogen auf enthaltene Salpetersäure) einsetzt.

14. Verfahren nach einem oder mehreren der Ansprüche 1 bis 13, dadurch gekennzeichnet, daß man den 3-Fluorbenzoesäurealkylester mit Nitriersäure bei 0 bis 20 °C umsetzt.

15. Verfahren nach einem oder mehreren der Ansprüche 1 bis 14, dadurch gekennzeichnet, daß man nach der Nitrierung das Reaktionsgemisch bei 40 bis 60 °C mit Wasser versetzt.

16. Verfahren nach einem oder mehreren der Ansprüche 1 bis 15, dadurch gekennzeichnet, daß man das nitrierte 5-Fluor-2-nitrobenzoesäurealkylester und 3-Flour-2-nitrobenzoesäurealkylester enthaltende Reaktionsprodukt durch Phasentrennung abtrennt und gegebenenfalls mit 40 bis 60 °C warmen Wasser wäscht.

17. Verfahren nach einem oder mehreren der Ansprüche 1 bis 16, dadurch gekennzeichnet, daß man aus dem nitrierten Reaktionsprodukt durch Schmelzkristallisation 3-Fluor-2-nitrobenzoesäurealkylester abtrennt und den verbleibenden 5-Fluor-2-nitrobenzoesäurealkylester gegebenenfalls durch Hydrolyse in der 5-Fluor-2-nitrobenzoesäure überführt.

18. Verfahren nach einem oder mehreren der Ansprüche 1 bis 17, dadurch gekennzeichnet, daß man das nitrierte Reaktionsprodukt in Gegenwart von 0,01 bis 0,1 Gew.-% eines Metalls der Platingruppe, bezogen auf nitriertes Reaktionsprodukt, mit Wasserstoff umsetzt.

19. Verfahren nach einem oder mehreren der Ansprüche 1 bis 18, dadurch gekennzeichnet, daß man das nitrierte Reaktionsprodukt, in Gegenwart eines Pd oder Pt und Schwefel enthaltenden Katalysators mit Wasserstoff umsetzt.

20. Verfahren nach einem oder mehreren der Ansprüche 1 bis 19, dadurch gekennzeichnet, daß man als Katalysator einen Pd oder Pt auf Aktivkohle Katalysator und untergeordnete Mengen einer Schwefelverbindung einsetzt.

21. Verfahren nach einem oder mehreren der Ansprüche 1 bis 20, dadurch gekennzeichnet, daß man als Katalysator einen sulfitierten oder sulfidierten Pd oder Pt auf Aktivkohle Katalysator und untergeordnete Mengen einer Schwefelverbindung einsetzt.

22. Verfahren nach einem oder mehreren der Ansprüche 1 bis 21, dadurch gekennzeichnet, daß man Schwefelverbindung und Platinmetall im Verhältnis (0,05 bis 40) zu 1, insbesondere (0,2 bis 10) zu 1, einsetzt.

23. Verfahren nach einem oder mehreren der Ansprüche 1 bis 22, dadurch gekennzeichnet, daß man als Schwefelverbindung Thioharnstoff, Dimethylsulfoxid, Thiophen und/oder ein Alkalisulfit einsetzt.

24. Verfahren nach einem oder mehreren der Ansprüche 1 bis 23, dadurch gekennzeichnet, daß man das nitrierte Reaktionsprodukt in Gegenwart eines inerten Lösungsmittels mit Wasserstoff umsetzt.

25. Verfahren nach einem oder mehreren der Ansprüche 1 bis 24, dadurch gekennzeichnet, daß man als inertes Lösungsmittel H₂O, einen Alkohol, Toluol, Xylol, Chlorbenzol und/oder Dichlorbenzol einsetzt.

26. Verfahren nach einem oder mehreren der Ansprüche 1 bis 25, dadurch gekennzeichnet, daß man als nitriertes Reaktionsprodukt eine wäßrige Lösung eines Salzes der isomerenreinen 5-Fluor-2-nitrobenzoesäure mit Wasserstoff umsetzt.

27. Verfahren nach einem oder mehreren der Ansprüche 1 bis 26, dadurch gekennzeichnet, daß man die Umsetzung mit Wasserstoff bei 0,5 bis 10, insbesondere 0,5 bis 5, bevorzugt 1,0 bis 3,0 MPa durchführt.

28. Verfahren nach einem oder mehreren der Ansprüche 1 bis 27, dadurch gekennzeichnet, daß man die Umsetzung mit Wasserstoff bei 60 bis 100 °C durchführt.

## Claims

1. A process for the preparation of 5-fluoroanthranilic alkyl esters and/or 5-fluoroanthranilic acid, which comprises dissolving a 3-fluorobenzoic alkyl ester in sulfuric acid and reacting the solution with nitrating acid at from -10 to 30°C, then adding water, separating off the nitrated reaction product and reacting it with hydrogen at from 50 to 120°C under elevated pressure in the presence of a catalyst comprising a metal of the platinum group and sulfur, and, if desired, removing 5-fluoroanthranilic alkyl esters by distillation and hydrolyzing them to give 5-fluoroanthranilic acid.

2. The process as claimed in claim 1, wherein a C₁-C₆-alkyl 3-fluorobenzoate is employed as 3-fluorobenzoic alkyl ester.

3. The process as claimed in claim 1 or 2, wherein methyl 3-fluorobenzoate, ethyl 3-fluorobenzoate, n-propyl 3-fluorobenzoate, isopropyl 3-fluorobenzoate, n-butyl 3-fluorobenzoate and/or isobutyl 3-fluorobenzoate is employed.

4. The process as claimed in one or more of claims 1 to 3, wherein methyl 3-fluorobenzoate and/or ethyl 3-fluorobenzoate is employed.

5. The process as claimed in one or more of claims 1 to 4, wherein methyl 3-fluorobenzoate is employed.

6. The process as claimed in one or more of claims 1 to 5, wherein the 3-fluorobenzoic alkyl ester is dissolved in H₂SO₄ at from 0 to 20°C.

7. The process as claimed in one or more of claims 1 to 6, wherein the 3-fluorobenzoic alkyl ester is dissolved in from 98 to 100% strength by weight H₂SO₄.

8. The process as claimed in one or more of claims 1 to 7, wherein the 3-fluorobenzoic alkyl ester is dissolved in concentrated H₂SO₄.

9. The process as claimed in one or more of claims 1 to 8, wherein the 3-fluorobenzoic alkyl ester is dissolved in from 2 to 10 parts by weight of H₂SO₄, based on 3-fluorobenzoic alkyl ester.

10. The process as claimed in one or more of claims 1 to 9, wherein the nitrating acid employed is a mixture of from 95 to 100% strength by weight HNO₃ and from 98 to 100% strength by weight H₂SO₄.

11. The process as claimed in one or more of claims 1 to 10, wherein the nitrating acid employed is a mixture consisting of 1 part by weight of HNO₃ and from 2 to 6 parts by weight of concentrated H₂SO₄.

12. The process as claimed in one or more of claims 1 to 11, wherein from 0.8 to 1.2 equivalents of nitrating acid (based on the nitric acid it contains) are employed per mole of 3-fluorobenzoic alkyl ester.

13. The process as claimed in one or more of claims 1 to 12, wherein from 0.95 to 1.05 equivalents of nitrating acid (based on the nitric acid it contains) are employed per mole of 3-fluorobenzoic alkyl ester.

14. The process as claimed in one or more of claims 1 to 13, wherein the 3-fluorobenzoic alkyl ester is reacted with nitrating acid at from 0 to 20°C.

15. The process as claimed in one or more of claims 1 to 14, wherein after nitration water is added to the reaction mixture at from 40 to 60°C.

16. The process as claimed in one or more of claims 1 to 15, wherein the reaction product comprising nitrated 5-fluoro-2-nitrobenzoic alkyl ester and 3-fluoro-2-nitrobenzoic alkyl ester is separated off by phase separation and, if desired, is washed with hot water at from 40 to 60°C.

17. The process as claimed in one or more of claims 1 to 16, wherein 3-fluoro-2-nitrobenzoic alkyl ester is separated off from the nitrated reaction product by melt crystallization and the 5-fluoro-2-nitrobenzoic alkyl ester which remains is converted, if desired, into 5-fluoro-2-nitrobenzoic acid by hydrolysis.

18. The process as claimed in one or more of claims 1 to 17, wherein the nitrated reaction product is reacted with hydrogen in the presence of from 0.01 to 0.1% by weight of a platinum group metal, based on nitrated reaction product.

19. The process as claimed in one or more of claims 1 to 18, wherein the nitrated reaction product is reacted with hydrogen in the presence of a catalyst containing Pd or Pt and sulfur.

20. The process as claimed in one or more of claims 1 to 19, wherein the catalyst employed comprises Pd or Pt on active charcoal with minor amounts of a sulfur compound.

21. The process as claimed in one or more of claims 1 to 20, wherein the catalyst employed comprises sulfited or sulfided Pd or Pt on active charcoal with minor amounts of a sulfur compound.

22. The process as claimed in one or more of claims 1 to 21, wherein sulfur compound and platinum metal are employed in a ratio of (from 0.05 to 40):1, in particular (from 0.2 to 10):1.

23. The process as claimed in one or more of claims 1 to 22, wherein the sulfur compound employed is thiourea, dimethyl sulfoxide, thiophene and/or an alkali metal sulfite.

24. The process as claimed in one or more of claims 1 to 23, wherein the nitrated reaction product is reacted with hydrogen in the presence of an inert solvent.

25. The process as claimed in one or more of claims 1 to 24, wherein the inert solvent employed is H₂O, an alcohol, toluene, xylene, chlorobenzene and/or dichlorobenzene.

26. The process as claimed in one or more of claims 1 to 25, wherein the nitrated reaction product which is reacted with hydrogen is an aqueous solution of a salt of isomerically pure 5-fluoro-2-nitrobenzoic acid.

27. The process as claimed in one or more of claims 1 to 26, wherein the reaction with hydrogen is carried out at from 0.5 to 10 MPa, in particular at from 0.5 to 5 MPa and preferably at from 1.0 to 3.0 MPa.

28. The process as claimed in one or more of claims 1 to 27, wherein the reaction with hydrogen is carried out at from 60 to 100°C.

## Revendications

1. Procédé pour la préparation d'esters d'alkyles de l'acide 5-fluoranthranilique et/ou de l'acide 5-fluoranthranilique caractérisé en ce que l'on dissout un ester d'alkyle de l'acide 3-fluorobenzoïque dans l'acide sulfurique et on fait réagir avec un acide de nitration à -10 à 30 °C, ensuite on ajoute de l'eau, on sépare le produit de réaction nitré et on fait réagir en présence d'un catalyseur, contenant un métal du groupe du platines et le soufre, avec l'hydrogène, à une température de 50 à 120 °C, sous une pression élevée et éventuellement, on sépare par distillation l'ester d'alkyle de l'acide 5-fluoranthranilique et on le saponifie pour obtenir l'acide 5-fluoranthranilique.

2. Procédé selon la revendication 1, caractérisé en ce que l'on utilise comme ester d'alkyle de l'acide 3-fluorobenzoïque un ester d'alkyle en C₁-C₆ de l'acide 3-fluorobenzoïque.

3. Procédé selon la revendication 1 ou 2, caractérisé en ce que l'on utilise l'ester méthylique de l'acide 3-fluorobenzoïque, l'ester éthylique de l'acide 3-fluoro-benzoïque, l'ester n-propylique de l'acide 3-fluorobenzoïque, l'ester i-propylique de l'acide 3-fluorobenzoïque, l'ester n-butylique de l'acide 3-fluorobenzoïque et/ou l'ester i-butylique de l'acide 3-fluorobenzoïque.

4. Procédé selon une ou plusieurs des revendications 1 à 3, caractérisé en ce que l'on utilise l'ester méthylique de l'acide 3-fluorobenzoïque et/ou l'ester éthylique de l'acide 3-fluorobenzoïque.

5. Procédé selon une ou plusieurs des revendications 1 à 4, caractérisé en ce que l'on utilise l'ester méthylique de l'acide 3-fluorobenzoïque.

6. Procédé selon une ou plusieurs des revendications 1 à 5, caractérisé en ce que l'on dissout l'ester d'alkyle de l'acide 3-fluorobenzoïque dans H₂SO₄ à une température de 0 à 20 °C.

7. Procédé selon une ou plusieurs des revendications 1 à 6, caractérisé en ce que l'on dissout l'ester d'alkyle de l'acide 3-fluorobenzoïque dans H₂SO₄ à 98 à 100 % en poids.

8. Procédé selon une ou plusieurs des revendications 1 à 7, caractérisé en ce que l'on dissout l'ester d'alkyle de l'acide 3-fluorobenzoïque dans l'acide H₂SO₄ concentré.

9. Procédé selon une ou plusieurs des revendications 1 à 8, caractérisé en ce que l'on dissout l'ester d'alkyle de l'acide 3-fluorobenzoïque dans 2 à 10 parties en poids de H₂SO₄ par rapport à l'ester d'alkyle de l'acide 3-fluorobenzoïque.

10. Procédé selon une ou plusieurs des revendications 1 à 9, caractérisé en ce que l'on utilise en tant qu'acide de nitration un mélange de HNO₃ à 95 à 100 % en poids et de H₂SO₄ à 98 à 100 % en poids.

11. Procédé selon une ou plusieurs des revendications 1 à 10, caractérisé en ce que l'on utilise en tant qu'acide de nitration un mélange composé d'une partie en poids de H₂NO₃ de 2 à 6 parties en poids de H₂SO₄ concentré.

12. Procédé selon une ou plusieurs des revendications 1 à 11, caractérisé en ce que l'on utilise pour 1 mole d'ester d'alkyle de l'acide 3-fluorobenzoïque 0,8 à 1,2 équivalent d'acide de nitration (par rapport à l'acide nitrique contenu).

13. Procédé selon une ou plusieurs des revendications 1 à 12, caractérisé en ce que l'on utilise par mole d'ester d'alkyle de l'acide 3-fluorobenzoïque 0,95 à 1,05 équivalent d'acide de nitration (par rapport à l'acide nitrique contenu).

14. Procédé selon une ou plusieurs des revendications 1 à 13, caractérisé en ce que l'on fait réagir l'ester d'alkyle de l'acide 3-fluorobenzoïque avec l'acide de nitration à une température de 0 à 20 °C.

15. Procédé selon une ou plusieurs des revendications 1 à 14, caractérisé en ce que l'on ajoute de l'eau au mélange réactionnel, à une température de 40 à 60 °C, après nitration.

16. Procédé selon une ou plusieurs des revendications 1 à 15, caractérisé en ce que l'on sépare le produit de réaction nitré contenant l'ester d'alkyle de l'acide 5-fluoro-2-nitrobenzoïque et l'ester d'alkyle de l'acide 3-fluoro-2-nitrobenzoïque par séparation de phases et éventuellement lave à l'eau chaude à 40 à 60 °C.

17. Procédé selon une ou plusieurs des revendications 1 à 16, caractérisé en ce que l'on sépare l'ester d'alkyle de l'acide 3-fluoro-2-nitrobenzoïque du produit réactionnel nitré par cristallisation par fusion et on transforme l'ester d'alkyle de l'acide 5-fluoro-2-nitrobenzoïque restant éventuellement par hydrolyse en l'acide 5-fluoro-2-nitrobenzoïque.

18. Procédé selon une ou plusieurs des revendications 1 à 17, caractérisé en ce que l'on fait réagir le produit réactionnel nitré avec l'hydrogène en présence de 0,01 à 0,1 % d'un métal du groupe du platine par rapport au produit réactionnel nitré.

19. Procédé selon une ou plusieurs des revendications 1 à 18, caractérisé en ce que l'on fait réagir le produit réactionnel nitré avec l'hydrogène en présence d'un catalyseur contenant Pd ou Pt et le soufre.

20. Procédé selon une ou plusieurs des revendications 1 à 19, caractérisé en ce que l'on utilise comme catalyseur un catalyseur contenant du Pd ou Pt sur du charbon actif et de faibles quantités d'un composés de soufre.

21. Procédé selon une ou plusieurs des revendications 1 à 20, caractérisé en ce que l'on utilise comme catalyseur un catalyseur sulfité ou sulfuré au Pd ou au Pt sur du charbon actif et contenant de faibles quantités en un composé de soufre.

22. Procédé selon une ou plusieurs des revendications 1 à 21, caractérisé en ce que l'on utilise le composé de soufre et le métal du groupe de platine dans un rapport de (0,05 à 40) à 1, plus particulièrement de (0,2 à 10) à 1.

23. Procédé selon une ou plusieurs des revendications 1 à 22, caractérisé en ce que l'on utilise comme composés de soufre la thiourée, le diméthylsulfoxyde, le thiophène et/ou un sulfite alcalin.

24. Procédé selon une ou plusieurs des revendications 1 à 23, caractérisé en ce que l'on fait réagir le produit réactionnel nitré avec l'hydrogène en présence d'un solvant inerte.

25. Procédé selon une ou plusieurs des revendications 1 à 24, caractérisé en ce que l'on utilise comme solvant inerte H₂O, un alcool, le toluène, le xylène, le chlorobenzène et/ou le dichlorobenzène.

26. Procédé selon une ou plusieurs des revendications 1 à 25, caractérisé en ce que l'on fait réagir avec l'hydrogène en tant que produit réactionnel nitré une solution aqueuse d'un sel de l'acide 5-fluoro-2-nitrobenzoïque sans isomères.

27. Procédé selon une ou plusieurs des revendications 1 à 26, caractérisé en ce que l'on met en oeuvre la réaction avec l'hydrogène à une pression de 0,5 à 10, plus particulièrement de 0,5 à 5, de manière préférée de 1,0 à 3,0 MPa.

28. Procédé selon une ou plusieurs des revendications 1 à 27, caractérisé en ce que l'on met en oeuvre la réaction avec l'hydrogène à une température de 60 à 100 °C.
